(19) Europäisches Patentamt

European Patent Office

Office européen des brevets

(11) **EP 0 965 946 B1**

(12) **FASCICULE DE BREVET EUROPEEN**

(45) Date de publication et mention
de la délivrance du brevet:
**01.10.2003   Bulletin 2003/40**

(51) Int Cl.⁷: **G06T 5/00**

(21) Numéro de dépôt: **99401453.8**

(22) Date de dépôt: **14.06.1999**

(54) **Procédé d'amelioration du rapport signal/bruit de l'image radiographique d'un objet en mouvement**

Verfahren zur Verbesserung des Signal-/Rauschverhältnisses im Röntgenbild eines bewegten Objektes

Method of improving signal-to-noise ratio in the radiographic image of a moving object

(84) Etats contractants désignés:
**DE GB IT**

(30) Priorité: **19.06.1998   FR 9807759**

(43) Date de publication de la demande:
**22.12.1999   Bulletin 1999/51**

(73) Titulaire: **COMMISSARIAT A L'ENERGIE ATOMIQUE
75752 Paris Cédex 15 (FR)**

(72) Inventeurs:
- **Rizo, Philippe
  38700 La Tronche (FR)**
- **Sauze, Rolland
  38000 Grenoble (FR)**

(74) Mandataire: **Audier, Philippe André et al
Brevalex,
3, rue du Docteur Lancereaux
75008 Paris (FR)**

(56) Documents cités:
**EP-A- 0 814 430**

- **OZKAN M K ET AL: "ADAPTIVE MOTION-COMPENSATED FILTERING OF NOISY IMAGE SEQUENCES" IEEE TRANSACTIONS ON CIRCUITS AND SYSTEMS FOR VIDEO TECHNOLOGY, vol. 3, no. 4, 1 août 1993 (1993-08-01), pages 277-290, XP000414654**

**Description**

Domaine technique et art antérieur

L'invention présentée concerne le problème du traitement de la radioscopie d'objets en mouvement.

En général, en radioscopie d'objets en mouvement, les temps de pose sont courts. Ceci conduit à des images très bruitées, car le nombre de photons qui ont pu être détectés durant la prise de vue est faible. Pour pallier à ce problème, deux techniques sont communément appliquées.

Lorsque le déplacement de l'objet est lent, la technique la plus simple consiste à sommer un petit nombre de radiographies successives (cumul glissant) en considérant que ces images sont identiques. Dans ce cas, on accepte une perte de résolution géométrique en contrepartie d'une diminution du bruit sur les images. Cette fonctionnalité est en général présente sur la plupart des systèmes de traitement d'images en temps réel. S'il arrive n photons par pixel sur le détecteur pour chaque radiographie, le rapport signal sur bruit de la radiographie sera : $\sqrt{n}$. Si on somme m images successives pour générer une image moins bruitée, le rapport signal/bruit de cette image sera : $\sqrt{n \cdot m}$. Par contre, on perd en résolution géométrique. Si l'objet se déplace de $\Delta x$ entre deux radiographies successives, la résolution du système sera de l'ordre de grandeur $m.\Delta x$.

Dans le cas d'objets en mouvement rapide, une autre technique consiste à sommer un petit nombre de radiographies successives en les décalant pour tenir compte du mouvement de l'objet. Cette technique est communément appelée TDI ("Transfers Delay Integration") dans la technologie des CCD. Au niveau instrumental, le TDI est en général mis en oeuvre au niveau du capteur. Dans le cas de capteurs CCD, les charges dans le détecteur se déplacent en même temps que les points de l'image. Ceci permet de réaliser la sommation en analogique, et de limiter, d'une part, le nombre de données à transférer et, d'autre part, le nombre d'opération à faire exécuter au CPU.

Cette technique est limitée à un petit nombre de contributions car, suivant leur disposition en profondeur dans l'objet, les projections des différentes structures de l'objet ne se déplacent pas à la même vitesse sur l'écran. Si on somme un grand nombre d'images successives, l'image résultante sera représentative d'un plan unique de l'objet. Afin d'illustrer ce problème, on peut considérer deux configurations.

Tout d'abord, dans le cas d'un objet fin par rapport à la distance source-détecteur, la position en profondeur de l'objet d'une structure n'influencera que très peu sur la position de la projection. On peut par conséquent appliquer l'opération de TDI sur l'ensemble du capteur, c'est-à-dire sur le nombre d'images qui correspond à la présence de la structure recherchée sur l'image. Dans cette configuration, le TDI est la méthode de référence.

Dans le cas d'un objet épais par rapport à la distance source-détecteur, la position de la projection d'une structure se déplace beaucoup en fonction de sa position en profondeur. Le TDI ne pourra donc pas facilement s'appliquer. Il aura tendance à donner une image nette du plan correspondant au décalage entre les images superposées, et une image floue des autres zones de l'objet. C'est notamment pour cette configuration qu'il a fallu développer un traitement spécifique qui est l'objet de la présente invention.

Dans le cas d'un objet en mouvement rapide, celui-ci se déplace beaucoup sur l'ensemble des images à combiner. Typiquement, dans l'application au contrôle des propulseurs de fusée, le nombre de radiographie qui peut être utilisé dans un cumul glissant est de l'ordre de 6. Au-dessus de ce nombre, la résolution géométrique de l'image résultat se dégrade très rapidement. De la même manière, l'utilisation du TDI est limitée à une vingtaine de radiographies dans lesquelles on n'utilise que la zone centrale du détecteur.

Le document EP-A-0 814 430 divulgue une autre solution. On produit une séquence restaurée d'images d'un objet en mouvement à partir de mesures bruitées. La dégradation à cause du bruit est plus importante dans le cas d'un objet en mouvement car un flou cinétique est alors produit. Le document propose de réduire le bruit en effectuant les étapes suivantes:

- obtention d'au moins une séquence d'images primitives du corps en mouvement
- évaluation d'une loi de mouvement de l'objet
- modification des images primitives pour obtenir des images restaurées en appliquant à la fois un critère d'adéquation des images restaurées aux mesures, un critère de régularisation spatiale des images restaurées et un critère de régularisation temporelle de la séquence des images restaurées en respectant la loi de mouvement du corps.

Exposé de l'invention

L'invention est définie par les revendications ajoutées.

Un but de l'invention est de fournir une image radiographique d'un objet épais en mouvement, qui combinerait un grand nombre d'images successives de cet objet durant son déplacement.

La méthode proposée consiste à améliorer le rapport signal sur bruit dans la radiographie au détriment d'une légère altération de la résolution géométrique de celle-ci. Selon le traitement mis en oeuvre, on génère une image qui soit la plus proche possible d'une radiographie obtenue avec la source à l'infini. C'est la radiographie qui serait obtenue si chaque rayon arrivait sur le détecteur suivant un plan perpendiculaire à celui-ci, appelé plan de projection. La méthode proposée utilise l'information contenue dans la zone du détecteur correspondant à la projection du plan de projection durant son déplacement.

La méthode proposée permet de combiner un grand nombre d'images radiographiques afin de limiter le bruit sur les images, tout en limitant la perte de résolution géométrique. L'autre avantage de cette méthode est qu'elle peut être mise en oeuvre sur la même architecture logicielle que la tomosynthèse. Par conséquent, cette méthode pourra être utilisée en temps réel en radioscopie.

L'invention a tout d'abord pour objet un procédé de radiographie selon la revendication 1.

L'étape de combinaison peut être une étape de sommation des valeurs correspondant à la projection du plan de projection.

Selon un mode de réalisation, l'objet se déplace en translation suivant une direction (D) et présente une épaisseur 1 suivant la direction du plan de projection, le nombre de pixels combinés pour engendrer un pixel de l'image résultat étant égal à :

$$N_{PS} = \sum_{i=1}^{i=m} \left( \frac{1. \, \Delta x. \, m}{(FG + 1)} \frac{FGd}{FG} \frac{1}{p} + 1 \right)$$

où p représente la taille d'un pixel, et où FG et FGd représentent respectivement la distance entre la source et l'objet et la distance entre la source et le détecteur, et où m est le nombre de positions successives de l'objet, séparées d'une distance $\Delta x$, pour lesquelles une image est réalisée.

Le mouvement de l'objet peut aussi être circulaire, le plan de projection étant un plan radial de l'objet.

Une table de correspondance peut être construite, qui associe chaque pixel de l'image radiographique à un pixel de la ligne de projection du plan de projection.

Ainsi, on peut définir une table Tab(k,Ie,Je)=Js qui associe le pixel (Ie,Je) de l'image N+k, où k est un nombre entier positif ou négatif avec le pixel Js de la projection du plan de projection.

L'invention a également pour objet un procédé de radiographie d'un objet en mouvement entre une source et un détecteur, comportant :

- la détermination d'une surface $S_0$, pour une position donnée de l'objet, cette surface se projetant, sur le détecteur, suivant une courbe $(C_1)$, sa projection se faisant selon une surface $S_P$ dans ses autres positions,
- la réalisation d'images radiographiques de l'objet au cours de son mouvement, à l'aide de la source et du détecteur,
- le traitement de la projection selon $(C_1)$, ce traitement comportant les étapes suivantes :

    a) discrétisation de la surface $S_0$, d'une part suivant les lignes 1 que parcourt le rayonnement de la source au détecteur, appelées lignes d'atténuation du rayonnement, d'autre part suivant des lignes de direction différente à celles des lignes 1, chaque point de la surface discrétisée étant repéré par ses coordonnées $(I_e, J_e)$,
    b) pour chaque position k de l'objet, calcul de la position $(I_d, J_d)$, dans $S_p$, de la projection sur le détecteur de chaque point repéré par ses coordonnées $(i_e, J_e)$, la valeur de la mesure en $(I_d, J_d)$ étant $f(I_d, J_d)$ sur $S_p$,
    c) calcul de la projection de $S_0$ sur $C_1$ qui est fonction de $I_d$, pour l'ensemble des positions de la surface $S_0$ lors du déplacement de l'objet, à partir des différentes positions k de la surface, et définition d'une fonction :

$$\sum_k \sum_{J_d} f_k(I_d, J_d) = projI_e \, ,$$

    d) parcourt de l'objet, en repérant la nouvelle position de $S_0$ dans l'objet qui s'est déplacé d'une position élémentaire, puis on revient en c),
    e) construction de la radiographie ligne par ligne, chacune étant mise en mémoire.

Brève description des figures

De toute façon, les caractéristiques et avantages de l'invention apparaîtront mieux à la lumière de la description qui va suivre. Cette description porte sur les exemples de réalisation, donnés à titre explicatif et non limitatif, en se référant à des dessins annexés sur lesquels :

- les figures 1 et 2 illustrent un mode de réalisation de l'invention,
- La figure 3 est un exemple de réalisation de l'invention, dans le cas d'un contrôle de masse de propulseurs, en tomographie radiale,
- la figure 4 représente schématiquement un fantôme de propulseur,
- les figures 5A et 5B sont des coupe suivant les structures, respectivement dans l'image en TDI (sur 35 radiographies) et dans l'image de radiographie radiale selon l'invention (sur 35 vues successives).

Description détaillée de modes de réalisation de l'invention

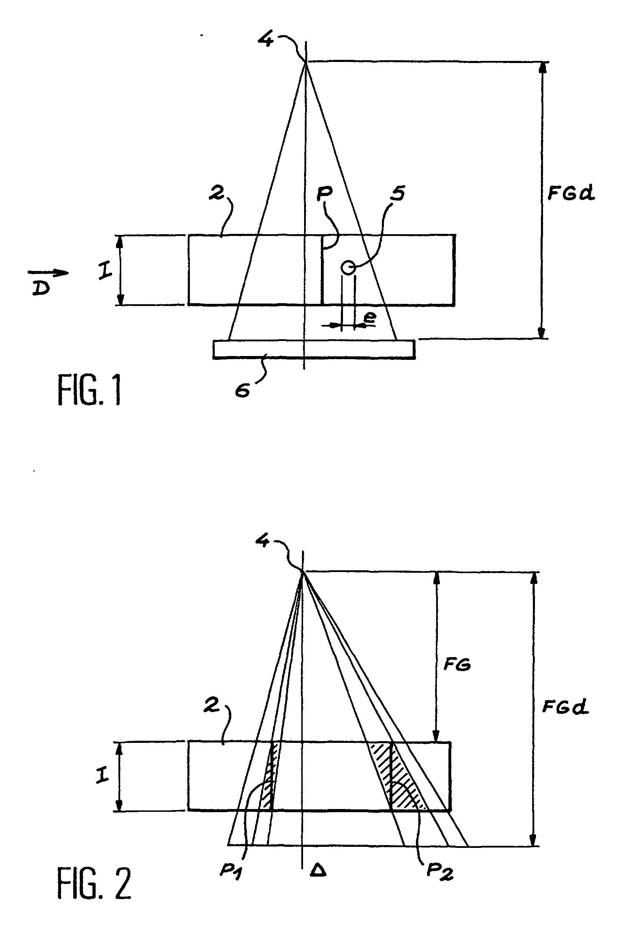

La figure 1 illustre un premier mode de réalisation de l'invention. On suppose un objet rectangulaire 2, d'épaisseur $\ell$, en translation entre une source 4 et un détecteur 6. On prend comme plan de projection P un plan perpendiculaire à la direction D du déplacement J de l'objet.

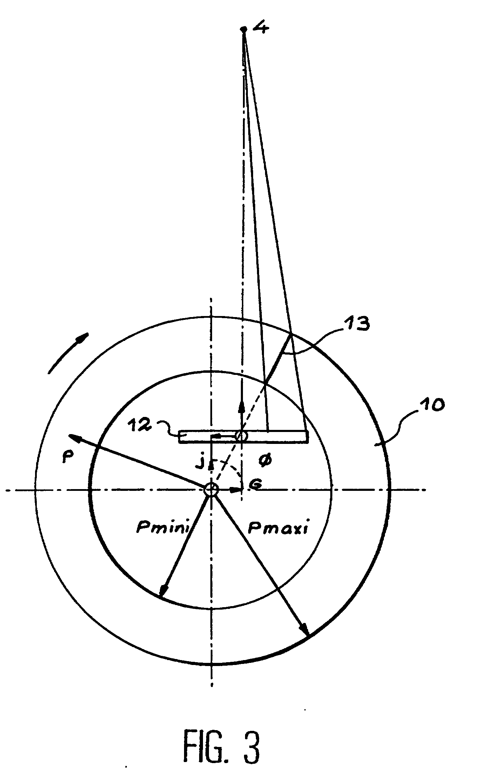

La figure 2 représente deux positions différentes $P_1$ et $P_2$ du plan de projection pendant le mouvement de l'objet 2.

Si on considère que l'atténuation de l'objet vaut 1 et que celle d'un défaut 5, de diamètre e, vaut 2, le contraste du défaut sur la radiographie (en densité) vaudra $\frac{e+\ell}{\ell}$, avec un rapport signal/bruit de $\sqrt{n}$, si n est le nombre de photon qui est détecté après traversée de l'objet.

Pour améliorer le rapport signal/bruit de l'image radiographique finale, on combine les différentes contributions des projections du plan de projection dans la série d'images. En fait, on somme, suivant la direction D du déplacement de l'objet, les valeurs correspondant à la projection du plan de projection. On génère ainsi une radiographie équivalente à une radiographie obtenue par un détecteur, ayant la forme de la plus petite projection du plan de projection et voyant défiler l'objet.

L'amélioration de rapport signal/bruit peut être calculée de la manière suivante. Soit x la distance entre le plan de projection $P_2$ et la perpendiculaire $\Delta$ au détecteur passant par la source 4, et a la largeur de la projection du plan de projection. Pour une valeur de x donnée, on a :

$$\frac{x}{FG+1} = \frac{y}{1} \text{ et } a = \frac{1.\, x}{(FG+1)} \frac{FGd}{FG}$$

où FGd représente la distance entre la source et le détecteur, et FG la distance entre la source et l'objet.

Quant a est plus petit que la taille p du pixel, on utilise les valeurs des lignes sur lesquelles se projette le plan de projection. Si le pas de déplacement en translation est $\Delta x$ et qu'on utilise m positions successives, le nombre de pixels combiné $N_{PS}$ pour générer un pixel résultat est :

$$N_{PS} = \sum_{i=1}^{i=m} \left( \frac{1.\, \Delta x.\, m}{(FG+1)} \frac{FGd}{FG} \frac{1}{p} + 1 \right)$$

$N_{PS}$ est largement supérieur à m ; par conséquent la statistique de mesure sur l'image reconstruite est toujours meilleure que la statistique qui serait obtenue par simple cumul glissant ou par TDI. Une estimation de ce gain de rapport signal/bruit sera présentée ci-dessous dans le cas du contrôle de propulseurs de fusée.

Selon un mode de réalisation, le traitement selon l'invention permet de construire une table de correspondance qui associe chaque pixel de l'image radiographique à un pixel de la ligne de projection d'un plan radial.

Une mise en oeuvre peut être la suivante.

Pour le plan radial se projetant orthogonalement sur l'image N, on définit une table Tab(k,Ie,Je)=Js qui associe le pixel (Ie,Je) de l'image N+k, k pouvant être négatif, avec le pixel Js de la projection du plan radial. De cette manière, on peut construire ligne par ligne une image radiographique dont chaque colonne est une projection d'un plan radial.

Cette approche a été mise en oeuvre dans le cadre du contrôle de masse de propulseurs (figure 3). Pour le contrôle (radiographie radiale), contrairement à radiographie tangentielle, un détecteur 12 est à l'intérieur du conduit d'un propulseur 10. La projection des défauts se déplace donc plus rapidement sur le détecteur que dans le cas de la radiographie tangentielle. Un schéma de l'installation est donné sur la figure 3.

Dans le cas de cette application, le plan de projection est un plan radial 13. On somme les contributions des projections de ce plan de projection sur le détecteur. Le propulseur se déplace par exemple dans le sens des aiguilles d'une montre (sens des $\phi$ croissants). Il pourrait aussi se déplacer en sens inverse.

La relation liant la largeur a de la projection sur le détecteur et la géométrie du système est la suivante :

$$a = FGd. \sin \phi \left( \frac{\rho_{max}}{FGd - \rho_{max} \cos \phi} - \frac{\rho_{min}}{FGd - \rho_{min} \cos \phi} \right)$$

$$a \approx FGd. \sin \phi \left( \frac{\rho_{max}}{FGd - \rho_{max}} - \frac{\rho_{min}}{FGd - \rho_{min}} \right)$$

où $\rho_{min}$ et $\rho_{max}$ désignent respectivement les diamètres intérieur et extérieur du propulseur 10. Comme dans les exemples précédents, 4 désigne la source de rayonnement et FGd la distance source-détecteur.

Si on considère que la source est loin du détecteur, on peut considérer que $\cos\phi \approx 1$. Si on veut garantir une résolution supérieure à 10 mm, c'est-à-dire qu'on ne prend pas en compte les contributions des points qui se trouvent à plus de 10 mm du plan de projection dont on calcule la projection, il faut limiter a. Avec a=10 mm, $\rho_{max}$=1500 mm, $\rho_{min}$=500 mm et FGd=3000 mm, on trouve que $\phi$=0,238°.

Par conséquent, on pourra effectuer le traitement en cumulant les projections pendant la rotation du propulseur sur un secteur angulaire de 2x0,238°=0,476°, en conservant une résolution géométrique supérieure à 10 mm. La largeur du pixel image étant d'environ 0,8 mm, chaque contribution correspond à la sommation de 10 lignes. D'autre part, entre deux images vidéo, on se déplace de 0,025°, par conséquent, on accumulera sur 20 positions angulaires. Les angles étant petits, la somme des contributions aura le même contraste que la projection brute, c'est-à-dire de l'ordre de $\frac{e + \ell}{\ell}$, avec une sommation d'environ 200 contributions identiques, c'est-à-dire avec un rapport signal/bruit amélioré d'un facteur 14.

Les performances du TDI et de la méthode que nous avons proposées ont été comparées sur une série de fantômes de tests avec des données simulées et expérimentales.

Lors des expérimentation pour un test de radiographie tangentielle, une série de mesure en géométrie radiale a été acquise sur un fantôme de propulseur en sable. Cette série de mesure a permis de réaliser une validation expérimentale préliminaire.

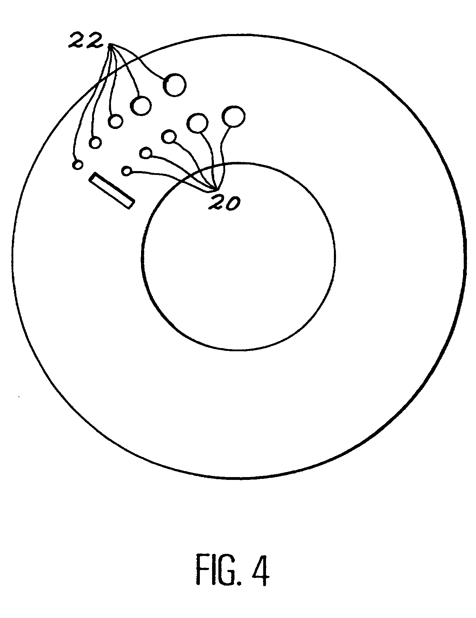

Le fantôme utilisé (figure 4) pour cette acquisition est constitué de deux séries 20, 22 de 5 inserts en polyéthylène de diamètres variables. Chacune des séries étant placée à un $\rho$ donné. Les deux séries d'inserts sont espacées de 20 cm.

La distance entre la source et le détecteur est de 4 m, et le détecteur mesure 30x40 cm ; le diamètre extérieur du propulseur est de 3 m et le diamètre intérieur de 1 m. Le propulseur est en rotation à 1° par seconde.

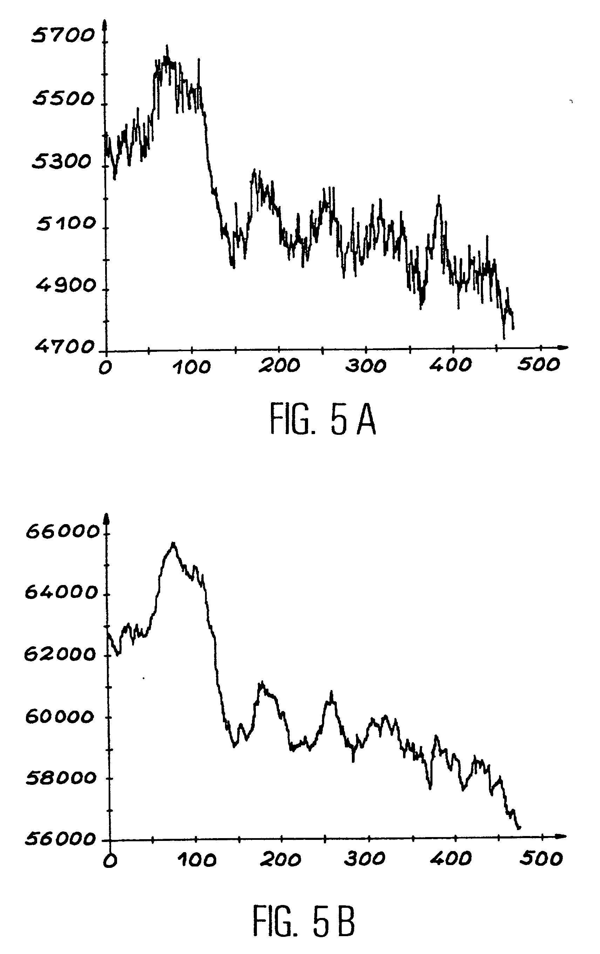

On a d'abord considéré que les défauts sont localisés a priori dans la zone centrale du propulseur et on a réalisé un traitement TDI focalisé sur ce rayon. On a limité le TDI à la combinaison de 35 images. La coupe résultante, suivant les structures, dans l'image TDI, sur 35 radiographies, est donnée en figure 5A.

On a aussi effectué le traitement de radiographie radiale selon l'invention, en combinant 35 images successives et en prenant, comme plans de projection, les plans radiaux. La coupe résultante, suivant les structures dans l'image de radiographie radiale sur 35 vues successives, est donnée en figure 5B.

On observe donc une nette amélioration du rapport signal/bruit avec la méthode proposée par rapport au TDI. La résolution géométrique entre les deux méthodes reste sensiblement la même.

Un procédé de radiographie selon l'invention, d'un objet en mouvement, à rapport signal/bruit amélioré, peut être le suivant.

Tout d'abord, on calibre la géométrie en fonction d'une première série de conditions d'acquisition, pour relier le déplacement de l'objet à son déplacement sur l'image. On acquiert donc des images de calibration (à l'aide, par exemple, de billes ou de grilles) . On obtient donc des paramètres caractéristiques de la géométrie d'acquisition, appelés paramètres intrinsèques, décrivant la géométrie du système de mesure, et des paramètres extrinsèques décrivant la trajectoire de l'objet dans le repère des mesures.

Ensuite, on définit des conditions d'acquisition, spécifiques aux conditions d'acquisition sans modification des paramètres géométriques.

A partir des paramètres géométriques et des conditions d'acquisition, on peut calculer des tables de mise en correspondance des pixels entre les images.

Une acquisition d'une série d'images de l'objet est ensuite réalisée.

Une surface de l'objet $S_0$ est sélectionnée, pour une position donnée de l'objet. Cette surface se projette sur le détecteur, suivant une courbe $C_1$ ; dans ses autres positions, sa projection se fait selon une surface $S_p$.

Puis un traitement de la projection selon C1 est réalisé :

a) discrétisation de la surface $S_0$, d'une part suivant les lignes 1 (appelées lignes d'atténuation du rayonnement) que parcourt le rayonnement, de la source au détecteur, d'autre part suivant des lignes de direction différente à celles des lignes 1 (par exemple perpendiculaires à 1), chaque point de la surface discrétisée étant repéré par ses coordonnées $(I_e, J_e)$,

b) pour chaque position k de l'objet, calcul de la position $(I_d, J_d)$, dans $S_p$, de la projection sur le détecteur de chaque point, repéré par ses coordonnées $(I_e, J_e)$, la valeur de la mesure en $(I_d, J_d)$ étant $f(I_d, J_d)$ sur $S_p$,

c) calcul de la projection de $S_0$ sur $C_1$, qui est fonction de $I_d$, pour l'ensemble des positions de la surface $S_0$, lors du déplacement de l'objet, à partir des différentes positions k de la surface, et définition d'une fonction :

$$\sum_k \sum_{J_d} f_k(I_d, J_d) = \texttt{projI}_e$$

d) parcourt de l'objet et en repérant la nouvelle position de $S_0$ dans l'objet qui s'est déplacé d'une position élémentaire, puis retour en c (les étapes a et b sont effectuées une fois pour toutes).

e) Construction de la radiographie ligne par ligne, chacune étant mise en mémoire.

Par exemple pour les rayons X, la géométrie d'acquisition comporte des paramètres extrinsèques et intrinsèques qui sont :

* pour les paramètres intrinsèques :

- la distance source-détecteur,
- le pas d'échantillonnage (appelé taille du pixel),
- la position de la projection orthogonale du point focal de la source sur le détecteur.

* pour les paramètres extrinsèques :

- les angles d'Euler, qui permettent de définir l'orientation de l'axe de rotation dans le repère source-détecteur,
- la position de l'origine et de l'axe dans le repère source-détecteur.

Les conditions d'acquisition sont :

- la vitesse du déplacement de l'objet et sa position (par exemple rayon, si on est dans le cas d'une géométrie circulaire ; ou abscisse curviligne sur une trajectoire quelconque). On peut en déduire une construction de la trajectoire de l'objet dans le repère source-détecteur,
- la trajectoire,
- la fréquence d'acquisitions,
- la taille des images.

Par exemple pour les rayons infrarouges, la caméra est la source de rayonnement, et on cherche la trajectoire de l'objet dans le repère de la caméra.

Tous les traitements de données présentés dans la présente demande peuvent être réalisés par un ordinateur spécialement programmé à cet effet. Les instructions de programme appropriées peuvent être stockées sur des disques magnétiques ou dans des unités conventionnelles RAM ou ROM.

**Revendications**

1. Procédé de radiographie d'un objet en mouvement entre une source (4) et un détecteur (6), comportant:

- la détermination d'un plan (P) dans l'objet, appelé plan de projection de l'objet, tel que, pour exactement une des positions de l'objet en mouvement, la projection de ce plan est une ligne dans l'image radiographique détectée,

- la réalisation d'images radiographiques de l'objet au cours de son mouvement, à l'aide de la source (4) et du détecteur (6), chaque image contenant une zone engendrée par des rayons traversant le plan de projection, appelée projection du plan de projection,

- la combinaison des contributions correspondantes des différentes projections du plan de projection dans la série d'images radiographiques obtenues, pour engendrer la représentation du plan de projection dans l'image résultante.

2. Procédé selon la revendication 1, l'étape de combinaison étant une étape de sommation des valeurs de pixels correspondant à la projection du plan de projection.

3. Procédé selon la revendication 2, l'objet se déplaçant en translation suivant une direction (D) et présentant une épaisseur 1 suivant la direction du plan de projection, le nombre de pixels combinés pour engendrer un pixel de l'image résultat étant égal à :

$$N_{PS} = \sum_{i=1}^{i=m} \left( \frac{1. \, \Delta x. \, m}{(FG + 1)} \frac{FGd}{FG} \frac{1}{p} + 1 \right)$$

où p représente la taille d'un pixel, et où FG et FGd représentent respectivement la distance entre la source (4) et l'objet (2) et la distance entre la source (4) et le détecteur (6), et où m est le nombre de positions successives de l'objet, séparées d'une distance $\Delta x$, pour lesquelles une image est réalisée.

4. Procédé selon l'une des revendications 1 ou 2, le mouvement de l'objet étant circulaire le plan de projection étant un plan radial de l'objet.

5. Procédé selon l'une des revendications 1 à 4, une table de correspondance étant construite, qui associe chaque pixel de l'image radiographique à un pixel de la ligne de projection du plan de projection.

6. Procédé selon la revendication 5, dans lequel on définit une table $Tab(k, I_e, J_e) = J_s$ qui associe le pixel $(I_e, J_e)$ de l'image N+k, où k est un nombre entier positif ou négatif, avec le pixel $J_s$ de la projection du plan de projection.

7. Procédé de radiographie d'un objet en mouvement entre une source (4) et un détecteur (6), comportant :

- la détermination d'une surface $S_0$, pour une position donnée de l'objet, cette surface se projetant, sur le détecteur, suivant une courbe $(C_1)$, sa projection se faisant selon une surface $S_P$ dans ses autres positions,
- la réalisation d'images radiographiques de l'objet au cours de son mouvement, à l'aide de la source (4) et du détecteur (6),
- le traitement de la projection selon $(C_1)$, ce traitement comportant les étapes suivantes :

a) discrétisation de la surface $S_0$, d'une part suivant les lignes 1 que parcourt le rayonnement de la source au détecteur, appelées lignes d'atténuation du rayonnement, d'autre part suivant des lignes de direction différente à celles des lignes 1, chaque point de la surface discrétisée étant repéré par ses coordonnées $(I_e, J_e)$,
b) pour chaque position k de l'objet, calcul de la position $(I_d, I_d)$, dans $S_p$, de la projection sur le détecteur de chaque point repéré par ses coordonnées $(I_e, J_e)$, la valeur de la mesure en $(I_d, J_d)$ étant $f(I_d, J_d)$ sur $S_p$,
c) calcul de la projection de $S_0$ sur $C_1$ qui est fonction de $I_d$, pour l'ensemble des positions de la surface $S_0$ lors du déplacement de l'objet, à partir des différentes positions k de la surface, et définition d'une fonction :

$$\sum_{k} \sum_{J_d} f_k(I_d, J_d) = proj I_e,$$

d) parcourt de l'objet, en repérant la nouvelle position de $S_0$ dans l'objet qui s'est déplacé d'une position élémentaire, puis retour à l'étape c),
e) construction de la radiographie ligne par ligne, chacune étant mise en mémoire.

**8.** Procédé selon la revendication 7, comportant en outre une étape de calibration de géométrie en fonction d'une première série de conditions d'acquisition, pour relier le déplacement de l'objet à son déplacement sur l'image, et obtention des paramètres caractéristiques de la géométrie d'acquisition, appelés paramètres intrinsèques, décrivant la géométrie du système de mesure, et des paramètres extrinsèques, décrivant la géométrie du système de mesure, et des paramètres extrinsèques décrivant la trajectoire de l'objet dans le repère des mesures.

**9.** Procédé selon l'une des revendications 7 ou 8 comportant en outre une étape de définition des conditions d'acquisition, spécifiques aux conditions d'acquisition sans modification des paramètres géométriques.


**Patentansprüche**

**1.** Verfahren zum Röntgen eines Objekts, das sich zwischen einer Quelle (4) und einem Detektor (6) bewegt, umfassend:

- die Festlegung einer Ebene (P) in dem Objekt, Projektionsebene des Objekts genannt, so dass für genau eine der Positionen des sich bewegenden Objekts die Projektion dieser Ebene eine Linie bzw. Zeile in dem detektierten Röntgenbild ist,
- die Realisierung von Röntgenbildern des Objekts im Laufe seiner Bewegung, mit Hilfe der Quelle (4) und des Detektors (6), wobei jedes Bild eine Projektion der Projektionsebene genannten Zone enthält, erzeugt durch Strahlen, die die Projektionsebene durchqueren,
- die Kombination der entsprechenden Beiträge der verschiedenen Projektionen der Projektionsebene in der Serie der erhaltenen Röntgenbilder, um die Darstellung der Projektionsebene in dem resultierenden Bild zu erzeugen.

**2.** Verfahren nach Anspruch 1, wobei der Kombinationsschritt ein Summierschritt der Werte von Pixeln ist, die der Projektion der Projektionsebene entsprechen.

**3.** Verfahren nach Anspruch 2, wobei das Objekt sich translatorisch entsprechend einer Richtung (D) bewegt und eine Dicke I gemäß der Richtung der Projektionsebene aufweist, und die Anzahl kombinierter Pixel zur Erzeugung eines Pixels des resultierenden Bildes nach folgender Formel berechnet wird:

$$N_{PS} = \sum_{i=1}^{i=m} \left( \frac{l.\Delta x.m}{(FG+l)} \frac{FGd}{FG} \frac{1}{p} + 1 \right)$$

wobei p die Größe eines Pixels darstellt und FG und FGd jeweils den Abstand zwischen der Quelle (4) und dem Objekt (2) und zwischen der Quelle (4) und dem Detektor (6) darstellen und m die Anzahl der aufeinanderfolgenden, durch einen Abstand $\Delta x$ getrennten Positionen des Objekts ist, für die ein Bild realisiert wird.

**4.** Verfahren nach einem der Ansprüche 1 oder 2, wobei die Bewegung des Objekts kreisförmig ist und die Projektionsebene eine radiale Ebene des Objekts ist.

**5.** Verfahren nach einem der Ansprüche 1 bis 4, wobei eine Korrespondenz- bzw. Entsprechungstabelle erstellt wird, die jedes Pixel des Röntgenbildes einem Pixel der Projektionslinie der Projektionsebene zuordnet.

**6.** Verfahren nach Anspruch 5, bei dem man eine Tabelle $Tab(k, I_e, J_e) = J_s$ definiert, die das Pixel $(I_e, J_e)$ des Bilds N+k, wobei k eine positive oder negative ganze Zahl ist, dem Pixel $J_s$ der Projektion der Projektionsebene zuordnet.

**7.** Verfahren zum Röntgen eines Objekts, das sich zwischen einer Quelle (4) und einem Detektor (6) bewegt, umfassend:

- die Festlegung einer Fläche $S_O$ für eine bestimmte Position des Objekts, wobei diese Fläche entsprechend

einer Kurve ($C_1$) auf den Detektor projiziert wird und ihre Projektion in ihren anderen Positionen entsprechend einer Fläche $S_P$ erfolgt,

- die Realisierung von Röntgenbildern des Objekts im Laufe seiner Bewegung mit Hilfe der Quelle (4) und dem Detektor (6),
- die Verarbeitung der Projektion gemäß ($C_1$), wobei diese Verarbeitung die folgenden Schritte umfasst:

a) Aufgliedern der Fläche $S_O$, einerseits gemäß den Linien I, die die Strahlung von der Quelle zum Detektor durchläuft, Strahlungsdämpfungslinien genannt, andererseits gemäß Linien mit Richtungen, die sich von denen den Linien I unterscheiden, wobei jeder Punkt der aufgegliederten Fläche mit seinen Koordinaten ($I_e$, $J_e$) versehen wird,

b) für jede Position k des Objekts die Berechnung der Position ($I_d$, $J_d$), in $S_P$, der Projektion auf den Detektor von jedem mit seinen Koordinaten ($I_e$, $J_e$) versehenen Punkt, wobei in $S_P$ der Wert der Messung in ($I_d$, $J_d$) $f(I_d, J_d)$ ist,

c) Berechnen der Projektion von $S_O$ in $C_1$, Funktion bzw. abhängig von $I_d$, für die Gesamtheit der Positionen der Fläche $S_O$ während der Bewegung des Objekts, ausgehend von verschiedenen Positionen k der Fläche, und Definition einer Funktion:

$$\sum_k \sum_{J_d} f_k(I_d, J_d) = \text{projI}_e \, ,$$

d) Durchlaufen bzw. Weiterbewegen des Objekts, wobei man die neue Position von $S_O$ in dem Objekt festlegt, das sich um eine elementare Position verschoben hat, dann Rückkehr zu Schritt c),

e) Erstellen des Röntgenbildes Zeile für Zeile, wobei jede abgespeichert wird.

8. Verfahren nach Anspruch 7 mit außerdem einem Schritt zur Geometrie-Kalibrierung in Abhängigkeit von einer ersten Serie von Erfassungsbedingungen, um die Verschiebung des Objekts mit seiner Verschiebung in dem Bild zu verknüpfen, und zur Erlangung der charakteristischen Parameter der Erfassungsgeometrie, die Geometrie des Messsystems beschreibende intrinsische Parameter, die Geometrie des Messsystems beschreibende extrinsische Parameter und den Weg des Objekts in dem Messbezugssystem beschreibende extrinsische Parameter genannt.

9. Verfahren nach einem der Ansprüche 7 oder 8, außerdem einen Schritt zur Definition der Erfassungsbedingungen umfassend, die spezifisch sind für die Erfassungsbedingungen ohne Modifikation der geometrischen Bedingungen.

**Claims**

1. Process for the radiography of a moving object between a source (4) and a detector (6) comprising:

- the determination of a plane (P) in the object, called object projection plane, such that for precisely one of the positions of the moving object, the projection of said plane is a line in the detected radiographic image,
- the production of radiographic images of the object during its movement with the aid of the source (4) and the detector (6), each image containing a zone produced by rays traversing the projection plane, called projection of the projection plane,
- the combination of the corresponding contributions of the different projections of the projection plane in the series of radiographic images obtained in order to produce the representation of the projection plane in the resulting image.

2. Process according to claim 1, the combination step being a step of summating pixel values corresponding to the projection of the projection plane.

3. Process according to claim 2, the object moving in translation in a direction (D) and having a thickness (1) in the direction of the projection plane, the number of combined pixels for producing a pixel of the resulting image being equal to:

$$N_{PS} = \sum_{i=1}^{i=m} \left( \frac{1.\Delta x.m}{(FG+1)} \frac{FGd}{FG} \frac{1}{P} \qquad +1 \right)$$

in which p represents the size of a pixel and where FG and FGd respectively represent the distance between the source (4) and the object (2) and the distance between the source (4) and the detector (6) and where m is the number of successive positions of the object separated by a distance ($\Delta x$) for which an image is created.

4. Process according to either of the claims 1 and 2, the movement of the object being circular, the projection plane being a radial plane of the object.

5. Process according to one of the claims 1 to 4, a correspondence table being built, which associates each pixel in the radiographic image with a pixel in the projection line of the projection plane.

6. Process according to claim 5, in which a table Tab(k, $I_e$, $J_e$) = Js is defined which associates pixel ($I_e$, $J_e$) in image N+k, where k is a positive or negative integer, with pixel Js in the projection of the projection plane.

7. Radiography process applicable for an object moving between a source (4) and a detector (6), comprising:

   - determination of an area $S_O$ for a given position of the object, this area being projected onto the detector along a curve ($C_1$), its projection taking place based on an area $S_P$ in its other positions,
   - production radiographic images of the object as it moves, using the source (4) and the detector (6),
   - processing of the projection along ($C_1$), this processing comprising the following steps:

     a) elementary breakdown of the area $S_O$, firstly along lines 1 along the direction of the radiation from the source to the detector, called radiation attenuation lines, and secondly along lines in a direction different from the direction of lines 1, each point on the meshed area being identified by its coordinates ($I_e$, $J_e$),
     b) for each position k of the object, calculation of the position ($I_d$, $J_d$), in $S_P$, of the projection onto the detector of each point identified by its coordinates ($I_e$, $J_e$), the value of the measurement in ($I_d$, $J_d$) being $f(I_d, J_d)$ on $S_P$,
     c) calculation of the projection of $S_O$ on $C_1$ which is a function of $I_d$, for all positions of the area $S_O$ while the object is moving, starting from the different positions k of the area, and definition of a function:

$$\sum_k \sum_{Jd} fk(Id, Jd) = projIe,$$

     d) movement of the object, identifying the new position of $S_O$ in the object which had moved by one elementary position, then return to step c),
     e) line by line construction of the radiograph, each line being stored.

8. Process according to claim 7, also comprising a step in which the geometry is calibrated as a function of a first series of acquisition conditions, to relate the movement of the object to its displacement on the image, and to obtain the characteristic parameters of the acquisition geometry called the intrinsic parameters describing the geometry of the measurement system, and extrinsic parameters describing the geometry of the measurement system and extrinsic parameters describing the trajectory of the object in the measurement coordinate system.

9. Process according to either of claims 7 or 8, also comprising a step in which the acquisition conditions are defined specific to the acquisition conditions without modification of the geometric parameters.

FIG. 1

FIG. 2

FIG. 3

FIG. 4

FIG. 5 A

FIG. 5 B